(19) **Europäisches Patentamt European Patent Office Office européen des brevets**

(11) **EP 0 669 303 B2**

(12) **NOUVEAU FASCICULE DE BREVET EUROPEEN**

(45) Date de publication et mention de la décision concernant l'opposition:
**08.08.2007 Bulletin 2007/32**

(45) Mention de la délivrance du brevet:
**29.10.1997 Bulletin 1997/44**

(21) Numéro de dépôt: **95400126.9**

(22) Date de dépôt: **23.01.1995**

(51) Int Cl.:
*C07C 17/38* (2006.01)     *C07C 19/08* (2006.01)

(54) **Procédé de séparation du fluorure d'hydrogène et du difluorométhane**

Verfahren zur Trennung von Fluorwasserstoff und Difluormethan

Process for the separation of hydrogen fluoride and difluoromethane

(84) Etats contractants désignés:
**BE DE ES FR GB GR IT NL**

(30) Priorité: **28.02.1994 FR 9402231**

(43) Date de publication de la demande:
**30.08.1995 Bulletin 1995/35**

(73) Titulaire: **Arkema France**
**92800 Puteaux (FR)**

(72) Inventeurs:
• **Galland, Jean-Michel**
 **F-69390 Vernaison (FR)**

• **Rouzies, Dominique**
 **F-69001 Lyon (FR)**

(74) Mandataire: **Pochart, François et al**
**Hirsch & Associés**
**58, Avenue Marceau**
**75008 Paris (FR)**

(56) Documents cités:
**WO-A-93/21140**

• **Complete English translation of WO 9321140 (D2)**

EP 0 669 303 B2

**Description**

L'invention concerne la séparation du fluorure d'hydrogène (HF) et du difluorométhane (F32) qui est un composé fluoré sans action sur la couche d'ozone et peut donc être utilisé comme produit de substitution des chlorofluorocarbures (CFC).

Le procédé selon l'invention est plus particulièrement destiné à la séparation de l'HF non transformé contenu dans les mélanges provenant de la fabrication du F32 par fluoration du chlorure de méthylène au moyen d'HF. Dans une telle fabrication, il est en effet économiquement nécessaire, d'une part, de récupérer sous forme anhydre l'HF non transformé pour le recycler au réacteur de fluoration et, d'autre part, de récupérer un F32 exempt le plus possible d'HF pour faciliter ensuite sa purification finale.

La plupart des hydrocarbures chlorofluorés ou fluorés forment avec l'HF des azéotropes ; la séparation de l'HF et de ces composés est donc difficile. Dans le but d'effectuer cette séparation, diverses techniques ont déjà été décrites. On peut citer par exemple :

- le brevet US 2 640 086 qui concerne la séparation de l'HF et du chlorodifluorométhane et utilise du chloroforme pour favoriser la décantation en deux phases, une phase riche en HF et une phase pauvre en HF ;
- le brevet US 3 873 629 relatif à un procédé continu pour la séparation d'HF et de chlorodifluorométhane et consistant à mettre en contact à contre-courant le mélange gazeux des deux constituants avec de l'acide sulfurique ;
- le brevet US 3 976 447 qui propose une séparation de l'HF d'effluents gazeux par absorption-désorption sur des particules de chlorure de calcium, baryum ou strontium ;
- le brevet US 4 209 470 qui décrit un procédé de séparation de l'HF de ses mélanges avec le 1-chloro-1,1-difluoro-éthane dans lequel, pour améliorer la décantation, on ajoute un liquide auxiliaire constitué totalement ou majoritairement par du 1,1-dichloro-1-fluoroéthane ;
- le brevet US 5 094 773 relatif à la séparation d'HF de ses mélanges avec le 2,2-dichloro-1,1,1-trifluoroéthane et/ou le 2chloro-1,1,1,2-tétrafluoroéthane par décantation et distillation;
- la demande de brevet EP 0 467 531 qui décrit un procédé de séparation du 1,1,1,2-tétrafluoroéthane de ses mélanges avec l'HF et/ou le 1-chloro-2,2-difluoroéthylène par double distillation avec ou sans décantation :
- la demande de brevet JP 5178 768 qui décrit la séparation du 1,1,1,2-tétrafluoroéthane de ses mélanges avec l'HF par double distillation.

Ces diverses techniques sont soit inéconomiques, soit inapplicables à la séparation de l'HF et du F32.

L'examen des données connues sur les variations de composition avec la pression des azéotropes entre l'HF et divers hydrocarbures chlorofluorés ou fluorés (voir tableau I suivant) montre que la teneur en HF de ces azéotropes varie relativement peu avec la pression. On constate en outre que, dans le cas du F22 et du F134a, la teneur en HF tend vers une valeur limite (respectivement 2,4 et 2,8 %) pour les pressions élevées.

**TABLEAU 1**

| Hydrocarbure chlorofluoré ou fluoré | Pression (bars absolus) | Teneur en HF de l'azéotrope (% poids) | Référence |
|---|---|---|---|
| Dichlorodifluorométhane (F12) | 2,8 | 4,5 | 1 |
| | 4,8 | 5,1 | 1 |
| | 7,8 | 8,0 | 1 |
| | 11,3 | 7,5 | 2 |
| Chlorodifluorométhane (F22) | 5,8 | 3 | 2 |
| | 11,3 | 2,7 | 2 |
| | 16,9 | 2,8 | 2 |
| | 25,2 | 2,4 | 3 |
| | 34,6 | 2,4 | 3 |
| 1,1,2-Trichloro-1,2,2-trifluoroéthane (F113) | 1,6 | 15,8 | 3 |
| | 9,5 | 20 | 3 |
| 1,2-Dichloro-1,1,2,2-tétrafluoroéthane (F114) | 2,9 | 9,5 | 3 |
| | 16,9 | 11,7 | 3 |

(suite)

| Hydrocarbure chlorofluoré ou fluoré | Pression (bars absolus) | Teneur en HF de l'azéotrope (% poids) | Référence |
|---|---|---|---|
| 1,1-Dichloro-2,2,2-trifluoroéthane (F123) | 1 | 17 | 4 |
| | 25 | 15,1 | 4 |
| | 40 | 14,9 | 4 |
| 1-Chloro-1,2,2,2-tétrafluoroéthane (F124) | 1 | 4,2 | 4 |
| | 25 | 5,8 | 4 |
| | 40 | 5,9 | 4 |
| 1,1,2,2-Tétrafluoroéthane (F134a) | 0,5 | 6,8 | 5 |
| | 0,5 | 1,2 | 6 |
| | 1 | 5,8 | 5 |
| | 1 | 1,7 | 6 |
| | 2 | 2,6 | 6 |
| | 3 | 4,1 | 5 |
| | 3 | 3,1 | 6 |
| | 4 | 3,6 | 6 |
| | 5 | 3,9 | 6 |
| | 6 | 3,3 | 5 |
| | 10 | 2,8 | 5 |
| | 16 | 2,8 | 5 |

*Références* **1** - M.A.ZAPOL'SKAYA et coll., Teor. Osnovy Khim. Tekh., 1975, pp 3-10 **2** - Azeotropic Data, 1973, 111, Horsley Lee H., American Chemical Society, Advances in Chemistry Series n°116, page 11 **3** - Données internes de la Demanderesse **4** - Brevet US 5 094 773 **5** - Demande de brevet EP 0 467 531 **6** - Demande de brevet JP 5 178 768

D'après la demande de brevet WO 93/21140 qui concerne la séparation de l'HF de ses mélanges avec le dichlorométhane, le chlorofluorométhane et/ou le difluorométhane, il est indiqué que la pression n'a pratiquement pas d'influence sur la composition de l'azéotrope HF-F32.

L'exemple 7 de ce document décrit un procédé dans lequel un mélange contenant 0,05g HF et 520g F32 était distillé utilisant une colonne de distillation sous reflux total en augmentant graduellement la température. Quand la pression de la colonne avait été augmentée à 22kg/cm$^2$ G et la température de la tête de colonne avait été augmentée à 40°C, la teneur du flux en HF était de 0,47% en poids. A une pression de 15kg/cm$^2$ G (15,7 bar absolus) et une température de 35°C en tête de colonne, le courant de flux contenait également 0,47% en poids de HF.

Contrairement à cet enseignement, il a maintenant été trouvé que, si l'HF et le F32 forment bien un azéotrope comme la plupart des hydrocarbures chlorofluorés ou fluorés, cet azéotrope HF-F32 se singularise (voir Tableau II ci-dessous) par une teneur en HF qui baisse considérablement lorsque la pression augmente et qui devient très faible (inférieure à 3000 ppm en poids) au-dessous de 20 bars absolus.

A partir des données du Tableau II, on peut représenter la pression Pa du mélange azéotropique (exprimée en bars absolus) en fonction de la teneur x en HF de ce mélange (exprimée en pour-cent poids) par la relation suivante :

$$Pa = 17 - 22,9 \, (x+0,821) \ln(x+0,608) + 56,6 \, [\ln(x+0,608)]^2$$

**TABLEAU II**

| AZEOTROPE HF-F32 | | |
|---|---|---|
| PRESSION (bars absolus) | TENEUR EN HF (% poids) | TEMPERATURE D'EBULLITION (°C) |
| 2,7 | 2,6 | -27 |
| 6 | 2,2 | -8,2 |
| 10 | 1 | 7,2 |
| 20 | 0.3 | 33,1 |
| 31 | 0,1 | 51,8 |

Ce comportement particulier de l'azéotrope HF-F32 était tout-à-fait inattendu et peut être mis à profit, conformément à la présente invention, pour réaliser des séparations industriellement efficaces de l'HF et du F32, notamment pour récupérer l'HF non transformé contenu dans les mélanges issus de la fabrication du F32 par fluoration du chlorure de méthylène au moyen d'HF et/ou pour obtenir un F32 quasi-exempt d'HF.

L'invention a pour objet un procédé de séparation du fluorure d'hydrogène (HF) et du difluorométhane (F32) d'un mélange gazeux issu d'une réaction de production de F32 par fluoration du chlorure de méthylène par de l'HF en phase liquide, ledit mélange gazeux contenant du F32, de l'HF, de l'HCl et divers autres produits, par rétrogradation ou condensation fractionnée sous une pression supérieure à 12 bars absolus, en une ou plusieurs étapes, caractérisé en ce qu'il comprend au moins une étape permettant d'obtenir un flux dont les teneurs en HF et F32 correspondent sensiblement à celles de la composition azéotropique, ladite étape étant effectuée, en fonction de l'objectif de séparation recherché, sous une pression choisie de façon telle que la pression partielle du mélange HF + F32 dudit flux (Pa exprimée en bars absolus) et la teneur en HF dudit mélange (x en pourcent poids) soient liées par la relation :

$$Pa = 17 - 22,9 \ (x+0,821)\ln(x+0,608) + 56,6 \ [\ln(x+0,608)]^2.$$

L'invention a aussi pour objet un procédé de séparation du fluorure d'hydrogène (HF) et du difluorométhane (F32) d'un mélange gazeux issu d'une réaction de production de F32 par fluoration du chlorure de méthylène par de l'HF en phase liquide, ledit mélange gazeux contenant du F32, de l'HF, de l'HCl et divers autres produits, par distillation sous une pression supérieure à 12 bars absolus, en une ou plusieurs étapes, caractérisé en ce qu'il comprend au moins une étape permettant d'obtenir un flux dont les teneurs en HF et F32 correspondent sensiblement à celles de la composition azéotropique, ladite étape étant effectuée, en fonction de l'objectif de séparation recherché, sous une pression choisie de façon telle que la pression partielle du mélange HF + F32 dudit flux (Pa exprimée en bars absolus) et la teneur en HF dudit mélange (x en pourcent poids) soient liées par la relation :

$$Pa = 17 - 22,9 \ (x+0,821)\ln(x+0,608) + 56,6 \ [\ln(x+0,608)]^2.$$

Selon un mode de réalisation, la séparation est effectuée sous une pression comprise entre 16 et 50 bars absolus.

Le procédé selon l'invention s'applique à la séparation de mélanges bruts de fabrication du F32. Cette fabrication peut être effectuée selon des procédés connus en soi qui peuvent être :

-    soit du type dit en phase liquide, généralement catalysé de manière homogène par des chlorofluorures d'antimoine,
-    soit du type dit en phase gazeuse, généralement catalysé de manière hétérogène par un catalyseur solide à base de divers métaux, par exemple du chrome.

Le mélange à séparer selon l'invention peut donc comprendre, en plus de l'HF et du F32, des proportions variables d'autres produits ou impuretés tels que, par exemple, de l'acide chlorhydrique, du chlorofluorométhane (F31), du chorodifluorométhane (F22), du trifluorométhane (F23), du chlore.... Le mélange à traiter peut être disponible à diverses pressions, être gazeux ou liquide, et contenir des proportions variables d'HF et de F32.

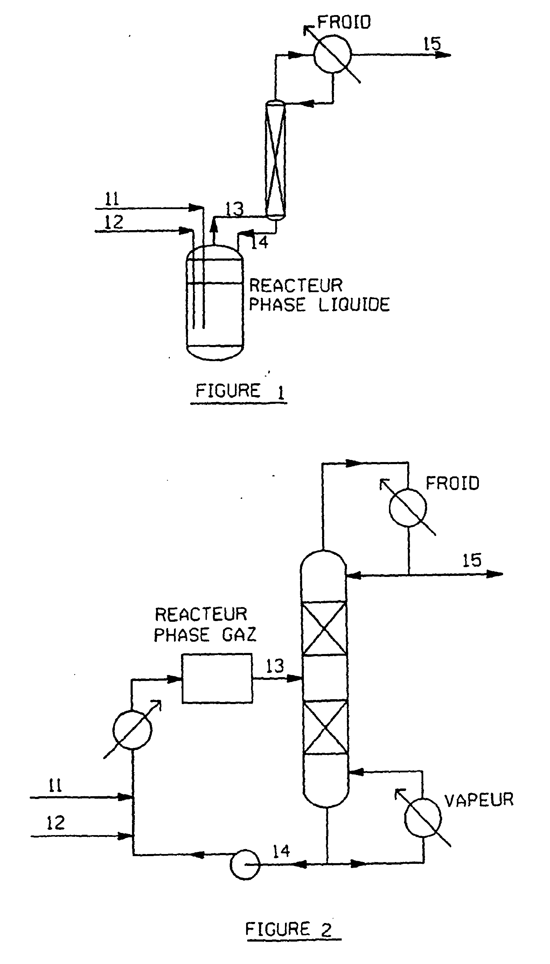

Le procédé selon l'invention peut être mis en oeuvre selon de nombreuses variantes, notamment celles correspondant aux schémas des Figures 1 à 2 annexées. Le choix par le fabricant de l'une ou l'autre variante dépendra de la nature du mélange à traiter et de l'objectif visé (récupération de l'essentiel de l'HF non transformé et/ou obtention d'un F32 quasi-exempt d'HF).

Un mode de mise en oeuvre, schématisé sur la Figure 1, concerne plus particulièrement la fabrication du F32 par réaction du chlorure de méthylène avec l'HF en phase liquide dans une installation classique comprenant un réacteur alimenté en HF et $CH_2Cl_2$ frais par les conduites 11 et 12 et un dispositif de rétrogradation. Les produits de réaction qui sortent du réacteur sous forme gazeuse et comportent de l'HCl, du F32, de l'HF et diverses impuretés, sont introduits par la conduite 13 dans une colonne de rétrogradation surmontée d'un condenseur. L'HCl, le F32 et une partie de l'HF non transformé sortent sous forme gazeuse en tête de colonne tandis que l'essentiel des produits organiques plus lourds et le reste de l'HF non transformé sont rétrogradés au réacteur par la conduite 14.

Dans le mode de mise en oeuvre selon la Figure 1, l'objectif de la séparation entre l'HF et le F32 effectuée dans le dispositif de rétrogradation est de minimiser le départ d'HF accompagnant le F32 produit et de recycler un maximum d'HF directement au réacteur. Pour atteindre l'objectif visé dans cette séparation, la mise en oeuvre du procédé selon l'invention consiste à conduire la rétrogradation à une pression supérieure à 12 bars absolus, de préférence comprise entre 16 et 50 bars absolus.

Malgré la présence d'HCl, la teneur maximale en HF du mélange HF-F32-HCl quittant le système réactionnel par la conduite 15 en aval du condenseur correspond sensiblement à la teneur en HF de l'azéotrope HF-F32 sous la pression partielle du mélange HF-F32, cette pression partielle étant égale à la différence entre la pression totale et la pression partielle de l'HCl. Comme la teneur en HF de l'azéotrope décroît fortement lorsque la pression croit, la teneur maximale en HF du mélange HF-F32-HCl quittant le système réactionnel décroît fortement lorsqu'on augmente la pression totale à laquelle on conduit la rétrogradation. Ainsi, en opérant à une pression supérieure à 12 bars absolus, la teneur en HF du flux sortant du système réactionnel par la conduite 15 est au plus de 2,5 % en poids, ce qui correspond largement à l'objectif de rétrograder au réacteur l'essentiel de l'HF non transformé.

Encore un autre mode de mise en oeuvre du procédé selon l'invention est schématisé sur la Figure 2 et concerne plus particulièrement la fabrication du F32 par réaction du chlorure de méthylène avec l'HF en phase gazeuse. En sortie du réacteur alimenté en HF et $CH_2Cl_2$ frais par les conduites 11 et 12, on obtient un mélange gazeux de F32, HCl, HF, F31 et $CH_2Cl_2$ qu'on introduit par la conduite 13 dans une colonne de distillation classique pour obtenir, d'une part, en tête un flux 15 constitué essentiellement par l'HCl et le F32 et, d'autre part, en pied un flux constitué essentiellement d'HF et d'organiques sous fluorés (F31, $CH_2Cl_2$) que l'on recycle au réacteur par la conduite 14.

Dans ce type de fabrication, l'objectif de la séparation entre l'HF et le F32 effectuée dans la colonne de distillation est de minimiser la teneur en HF du flux 15 de façon à recycler en réaction l'essentiel de l'HF non transformé. Pour atteindre cet objectif, la mise en oeuvre du procédé selon l'invention consiste à conduire la distillation à une pression supérieure à 12 bars absolus, de préférence comprise entre 16 et 50 bars absolus.

Comme dans le cas de la fabrication en phase liquide, la teneur maximale en HF du flux 15 quittant le système réactionnel correspond sensiblement à la teneur en HF de l'azéotrope HF-F32 sous la pression partielle du mélange HF-F32 et décroît fortement avec l'augmentation de la pression opératoire.

Les exemples suivants illustrent l'invention sans la limiter. Les pourcentages indiqués sont exprimés en poids.

## EXEMPLE 1

Les caractéristiques de l'azéotrope HF-F32 indiquées dans le Tableau II précédent ont été déterminées par mesure de la composition de la phase gaz de différents mélanges d'HF et de F32 après leur mise en équilibre sous différentes pressions.

La mise en équilibre est réalisée dans un récipient en inox de 102 ml, équipé d'un tube plongeur et d'une sortie phase gaz. Le mélange HF-F32 étudié est préparé par pesée en introduisant séparément l'HF et le F32. Dans le récipient préalablement placé dans un bain thermostaté à -20°C et mis sous vide, on introduit d'abord l'HF, puis on pèse le récipient et répète l'opération pour l'introduction du F32, le volume total de liquide dans le récipient représentant 80 ml. Après remplissage, le récipient est fermé, puis replacé dans le bain thermostaté et porté à la pression étudiée. La mesure de température et l'analyse du mélange sont effectuées 12 heures après la stabilisation de la pression et de la température.

L'analyse est réalisée par chromatographie gazeuse sur un prélèvement de la phase gaz du mélange à l'équilibre, chaque analyse étant effectuée trois fois avant de porter le mélange à une pression supérieure ou de recharger des produits.

Le tableau suivant rassemble la série des mesures effectuées. Pour chaque pression, on dispose de plusieurs mesures qui encadrent et donc permettent de déterminer l'azéotrope.

| PRESSION (bars absolus) | TEMPERATURE (°C) | Teneur en F32 (% poids) | |
|---|---|---|---|
| | | phase liquide | phase vapeur |
| 2,7 azéotrope | -26,8 | 97,10 | 97,55 |
| | -27,1 | 98,05 | 97,60 |

(suite)

| PRESSION (bars absolus) | TEMPERATURE (°C) | Teneur en F32 (% poids) | |
|---|---|---|---|
| | | phase liquide | phase vapeur |
| | -27,2 | 99,47 | 98,61 |
| | -27,3 | 99,88 | 99,73 |
| | -27 | 97,40 | 97,40 |
| 6 azéotrope | 23,3 | 20,71 | 89,63 |
| | 2,7 | 49,24 | 96,01 |
| | -3,2 | 79,33 | 97,21 |
| | -6,7 | 91,27 | 97,70 |
| | -8,2 | 97,86 | 97,86 |
| | -8,6 | 99,02 | 98,58 |
| | -8,8 | 99,38 | 99,10 |
| | -8,2 | 97,80 | 97,80 |
| 10 azéotrope | 45,5 | 20.71 | 90,13 |
| | 20,2 | 49,24 | 96,82 |
| | 12,6 | 79,33 | 97,69 |
| | 9,8 | 91,27 | 98,28 |
| | 9,1 | 97,86 | 98,72 |
| | 7,2 | 99,02 | 98,98 |
| | 7,4 | 99,38 | 99,26 |
| | 7,2 | 99,00 | 99,00 |
| 20 azéotrope | 76,6 | 20,71 | 90,75 |
| | 53,4 | 49,24 | 97,27 |
| | 43.8 | 79.33 | 98,47 |
| | 35,6 | 91,27 | 99,13 |
| | 34,5 | 97,86 | 99,65 |
| | 33,5 | 99,02 | 99,66 |
| | 33,2 | 99,38 | 99,68 |
| | 33,1 | 99,70 | 99,70 |
| 31 azéotrope | 52,2 | 98,05 | 99,73 |
| | 52 | 99,47 | 99.76 |
| | 51,9 | 99,87 | 99,89 |
| | 51,8 | 99,90 | 99,90 |

## EXEMPLE 2

On opère sous 20 bars absolus conformément au schéma de la Figure 1. La colonne de rétrogradation comporte 6 plateaux théoriques.

Le tableau suivant résume les conditions opératoires et les résultats obtenus.

| | ALIMENTATION 11 | ALIMENTATION 12 | SORTIE 15 |
|---|---|---|---|
| % CH$_2$Cl$_2$ | 100 | 0 | 0 |
| % HF | 0 | 100 | 0,8 |
| % F32 | 0 | 0 | 41,3 |
| % HCl | 0 | 0 | 57,9 |
| Pression (bars absolus) | 20 | 20 | 20 |
| Température (°C) | 20 | 20 | -5 |

Le taux de transformation global de l'HF dans ce système réactionnel est de 97,5 %.

## EXEMPLE 3

Dans le dispositif selon le schéma de la Figure 2, on effectue la fluoration du chlorure de méthylène en phase gazeuse sous 20 bars absolus. Le réacteur est alimenté en continu par les réactifs frais (HF et F32) et par le recyclage du flux 14 provenant du pied de la colonne à distiller qui comporte 13 plateaux théoriques.

Le tableau suivant résume les conditions opératoires et les résultats obtenus.

|  | ALIMENTATION 11 | ALIMENTATION 12 | FLUX 13 | FLUX 14 (recyclé) | SORTIE 15 |
|---|---|---|---|---|---|
| % $CH_2Cl_2$ | 100 | 0 | 15,3 | 28,4 | 0 |
| % HF | 0 | 100 | 29,1 | 53,2 | 0,8 |
| %F31 | 0 | 0 | 9,9 | 18,4 | 0 |
| % F32 | 0 | 0 | 19 | 0 | 41,3 |
| % HCl | 0 | 0 | 26,7 | 0 | 57,9 |
| Pression (bars absolus) | 20 | 20 | 20 | 20 | 20 |
| Température (°C) | 20 | 20 | 50 | 90 | -5 |

## Revendications

1. Procédé de séparation du fluorure d'hydrogène (HF) et du difluorométhane (F32) d'un mélange gazeux issu d'une réaction de production de F32 par fluoration du chlorure de méthylène par de l'HF en phase liquide, ledit mélange gazeux contenant du F32, de l'HF, de l'HCl et divers autres produits, par rétrogradation ou condensation fractionnée sous une pression supérieure à 12 bars absolus, en une ou plusieurs étapes, **caractérisé en ce qu'**il comprend au moins une étape permettant d'obtenir un flux dont les teneurs en HF et F32 correspondent sensiblement à celles de la composition azéotropique, ladite étape étant effectuée, en fonction de l'objectif de séparation recherché, sous une pression choisie de façon telle que la pression partielle du mélange HF + F32 dudit flux (Pa exprimée en bars absolus) et la teneur en HF dudit mélange (x en pourcent poids) soient liées par la relation :

$$Pa = 17 - 22,9 \ (x+0,821)\ln(x+0,608) \ + \ 56,6 \ [\ln(x+0,608)]^2.$$

2. Procédé de séparation du fluorure d'hydrogène (HF) et du difluorométhane (F32) d'un mélange gazeux issu d'une réaction de production de F32 par fluoration du chlorure de méthylène par de l'HF en phase liquide, ledit mélange gazeux contenant du F32, de l'HF, de l'HCl et divers autres produits, par distillation sous une pression supérieure à 12 bars absolus, en une ou plusieurs étapes, **caractérisé en ce qu'**il comprend au moins une étape permettant d'obtenir un flux dont les teneurs en HF et F32 correspondent sensiblement à celles de la composition azéotropique, ladite étape étant effectuée, en fonction de l'objectif de séparation recherché, sous une pression choisie de façon telle que la pression partielle du mélange HF + F32 dudit flux (Pa exprimée en bars absolus) et la teneur en HF dudit mélange (x en pourcent poids) soient liées par la relation :

$$Pa = 17 - 22,9 \ (x+0,821)\ln(x+0,608) \ + \ 56,6 \ [\ln(x+0,608)]^2.$$

3. Procédé selon la revendication 1 ou 2, dans lequel la séparation est effectuée sous une pression comprise entre 16 et 50 bars absolus.

4. Procédé selon la revendication 3, dans lequel la seconde distillation est effectuée sous une pression inférieure à 10 bars absolus.

5. Procédé selon la revendication 1 pour la séparation d'un mélange gazeux issu d'une réaction de production de F32 par fluoration du chlorure de méthylène par de l'HF en phase liquide, **caractérisé en ce que** ledit mélange gazeux contenant du F32, de l'HF, de l'HCl et divers autres produits est soumis à une opération de rétrogradation ou de condensation fractionnée sous une pression supérieure à 12 bars absolus.

6. Procédé selon la revendication 1 pour la séparation d'un mélange gazeux issu d'une réaction de production de F32 par fluoration du chlorure de méthylène par de l'HF en phase gazeuse, **caractérisé en ce que** ledit mélange gazeux contenant du F32, de l'HF, de l'HCl et divers autres produits est soumis à une distillation sous une pression supérieure à 12 bars absolus.

**7.** Procédé selon la revendication 5 ou 6, dans lequel la séparation est effectuée sous une pression comprise entre 16 et 50 bars absolus.

**Claims**

**1.** Process for the separation of hydrogen fluoride (HF) and of difluoromethane (F32) from a gaseous mixture originating from a reaction for producing F32 by fluorination of methylene chloride with HF in liquid phase, the said gaseous mixture containing F32, HF, HCl and various other products, by retrogradation or fractional condensation at a pressure above 12 bars absolute, in one or several stages, **characterized in that** said process includes at least one stage making it possible to obtain a stream whose HF and F32 contents correspond substantially to those of the la azeotropic composition, the said stage being performed, as a function of the intended separation objective, at a pressure chosen so that the partial pressure of the HF + F32 mixture of the said stream (Pa expressed in bars absolute) and the HF content of the said mixture (x in per cent by weight) are linked by the relationship:

$$Pa = 17 - 22,9\,(x{+}0,821)\ln(x{+}0,608) + 56,6\,[\ln(x{+}0,608)]^2.$$

**2.** Process for the separation of hydrogen fluoride (HF) and of difluoromethane (F32) from a gaseous mixture originating from a reaction for producing F32 by fluorination of methylene chloride with HF in liquid phase, the said gaseous mixture containing F32, HF, HCl and various other products, by distillation at a pressure above 12 bars absolute, in one or several stages, **characterized in that** said process includes at least one stage making it possible to obtain a stream whose HF and F32 contents correspond substantially to those of the la azeotropic composition, the said stage being performed, as a function of the intended separation objective, at a pressure chosen so that the partial pressure of the HF + F32 mixture of the said stream (Pa expressed in bars absolute) and the HF content of the said mixture (x in per cent by weight) are linked by the relationship:

$$Pa = 17 - 22,9\,(x{+}0,821)\ln(x{+}0,608) + 56,6\,[\ln(x{+}0,608)]^2.$$

**3.** Process according to Claim 1 or 2, in which the separation is performed under a pressure comprised between 16 and 50 bars absolute.

**4.** Process according to Claim 3, in which the second distillation is performed at a pressure below 10 bars absolute.

**5.** Process according to Claim 1, for the separation of a gaseous mixture originating from a reaction for producing F32 by fluorination of methylene chloride with HF in liquid phase, **characterized in that** the said gaseous mixture containing G32, HF, HCl and various other products is subjected to a fractional return or condensation operation at a pressure above 12 bars absolute.

**6.** Process according to Claim 1, for the separation of a gaseous mixture originating from a reaction for producing F32 by fluorination of methylene chloride with HF in gaseous phase, **characterized in that** the said gaseous mixture containing F32, HF, HCl and various other products is subjected to a distillation at a pressure above 12 bars absolute.

**7.** Process according to Claim 5 or 6, in which the separation is performed at a pressure of between 16 and 50 bars absolute.

**Patentansprüche**

**1.** Verfahren zur Trennung von Fluorwassertoff (HF) und Difluoromethan (F32) einer gasförmigen Mischung die aus einer Reaktion zur Herstellung von F32 mittels Fluorierung von Methylenchlorid durch HF in flüssiger Phase stammt, wo die gasförmige Mischung die F32, HF, HCl und verschiedene andere Produkte enthält, in einem oder mehreren Verfahrensschritten, einer Retrogradation oder einer fraktionierten Kondensation unter einem Druck von mehr als 12 bar absolut unterworfen wird, **dadurch gekennzeichnet daß** es mindestens einen Verfahrensschritt umfaßt, der es ermöglicht, einen Fluß zu erhalten, dessen Gehalt an HF und F32 in etwa den jeweiligen Gehalten der azeotropen Zusammensetzung entspricht, wobei dieser Verfahrensschritt in Abhängigkeit von der gewünschten Trennung unter einem Druck durchgeführt wird, der derart ausgewählt ist, daß der Partialdruck der Mischung HF + F32 dieses Flusses (Pa berechnet in bar absolut) und der Gehalt an HF dieser Mischung (x in Gewichtsprozent) durch die folgende Beziehung miteinander verbunden sind:

$$Pa = 17 - 22,9\,(x+0,821)\ln(x+0,608) + 56,6\,[\ln(x+0,608)]^2.$$

2. Verfahren zur Trennung von Fluorwassertoff (HF) und Difluoromethan (F32) einer gasförmigen Mischung die aus einer Reaktion zur Herstellung von F32 mittels Fluorierung von Methylenchlorid durch HF in flüssiger Phase stammt, wo die gasförmige Mischung die F32, HF, HCl und verschiedene andere Produkte enthält, in einem oder mehreren Verfahrensschritten, einer Destillation unter einem Druck von mehr als 12 bar absolut unterworfen wird, **dadurch gekennzeichnet daß** es mindestens einen Verfahrensschritt umfaßt, der es ermöglicht, einen Fluß zu erhalten, dessen Gehalt an HF und F32 in etwa den jeweiligen Gehalten der azeotropen Zusammensetzung entspricht, wobei dieser Verfahrensschritt in Abhängigkeit von der gewünschten Trennung unter einem Druck durchgeführt wird, der derart ausgewählt ist, daß der Partialdruck der Mischung HF + F32 dieses Flusses (Pa berechnet in bar absolut) und der Gehalt an HF dieser Mischung (x in Gewichtsprozent) durch die folgende Beziehung miteinander verbunden sind:

$$Pa = 17 - 22,9\,(x+0,821)\ln(x+0,608) + 56,6\,[\ln(x+0,608)]^2.$$

3. Verfahren nach Anspruch 1 oder 2, bei dem die Abtrennung unter einem Druck zwischen 16 und 50 bar absolut durchgeführt wird.

4. Verfahren nach Anspruch 3, bei dem die zweite Destillation unter einem Druck unterhalb von 10 bar absolut durchgeführt wird.

5. Verfahren nach Anspruch 1 zur Trennung einer gasförmigen Mischung, die aus einer Reaktion von F32 mittels Fluorierung von Methylenchlorid durch HF in flüssiger Phase stammt, **dadurch gekennzeichnet, daß** die gasförmige Mischung, die F32, HF, HCl und verschiedene andere Produkte enthält, einer Retrogradation oder einer fraktionierten Kondensation unter einem Druck von mehr als 12 bar absolut unterworfen wird.

6. Verfahren nach Anspruch 1 zur Abtrennung einer gasförmigen Mischung, die aus einer Reaktion zur Herstellung von F32 mittels Fluorierung von Methylenchlorid mit HF in der Gasphase stammt, **dadurch gekennzeichnet, daß** die gasförmige Mischung, die F32, HF, HCl und verschiedene andere Produkte enthält, einer Destillation unter einem Druck von mehr als 12 bar absolut unterworfen wird.

7. Verfahren nach Anspruch 5 oder 6, bei dem die Abtrennung unter einem Druck zwischen 16 und 50 bar absolut durchgeführt wird.

FROID

15

11

12

13

14

REACTEUR
PHASE LIQUIDE

FIGURE 1

FROID

15

REACTEUR
PHASE GAZ

13

11

12

14

VAPEUR

FIGURE 2